Europäisches Patentamt

European Patent Office

Office européen des brevets

(19)

(11) Publication number: **0 396 100**
**A1**

## (12) EUROPEAN PATENT APPLICATION

(21) Application number: **90108267.7**

(22) Date of filing: **30.04.90**

(51) Int. Cl.⁵: **C07D 211/22, C07B 63/00**

(30) Priority: **01.05.89 US 345799**

(43) Date of publication of application:
**07.11.90 Bulletin 90/45**

(84) Designated Contracting States:
**AT BE CH DE DK ES FR GB GR IT LI LU NL SE**

(71) Applicant: **MERRELL DOW PHARMACEUTICALS INC.**
**2110 East Galbraith Road**
**Cincinnati Ohio 45215-6300(US)**

(72) Inventor: **McCarty, Frederick J.**
**7141 Juniperview Lane**
**Cincinnati, Ohio 45243(US)**

(74) Representative: **Vossius & Partner**
**Siebertstrasse 4 P.O. Box 86 07 67**
**D-8000 München 86(DE)**

(54) **Process for the preparation of the high melting polymorphic form of terfenadine.**

(57) A process for the preparation of the high-melting polymorphic form of terfenadine which comprises dissolving terfenadine in acetone, diluting the solution with water and recovering the crystalized product.

EP 0 396 100 A1

# PROCESS FOR THE PREPARATION OF THE HIGH MELTING POLYMORPHIC FORM OF TERFENADINE

Terfenadine, 1-(p-tert-butylphenyl)-4-[4'-(alpha-hydroxydiphenylmethyl)-1'-piperidinyl]butanol, is a non-sedating antihistamine. Previous crystallization procedures for producing terfenadine result in a product which, although analytically pure, nevertheless has a widely variable melting point. Recently, it was discovered that solid terfenadine exists in two distinct crystalline or polymorphic forms, each form having a different melting point. It was further discovered that terfenadine crystallized by the prior art processes varied widely in polymorphic composition and that this polymorphic variation accounted for the observed variation of the melting point of terfenadine.

In an attempt to attain a material with a controlled set of physical properties, which facilitates quality control functions associated with large scale production of terfenadine, a process has recently been developed wherein both polymorphic forms of terfenadine can be produced. (See, U.S. Patent No. 4,742,175, issued May 3, 1988.) This patent concerns mainly the production of the high-melting polymorphic form (Form I) of terfenadine where the terfenadine is digested at reflux in a water-miscible alkanol. It additionally claims a process for producing the low-melting polymorphic form of terfenadine (Form II) by dissolving terfenadine in toluene or acetone at about 0°C to about 35°C and allowing the mixture to slowly evaporate over a two-day period.

Additionally, there is a prior pending EPO application. yet unpublished (EP 90 10 3752.3 claiming priority from U.S. Application No. 7/316,902, filed February 28, 1989) disclosing a process for producing the low melting polymorphic form of terfenadine (Form II) which involves controlled heating and subsequent cooling of a solution of terfenadine in an appropriate solvent. Acetone is one of the solvents disclosed in thb process of that application.

This invention relates specifically to a process for producing the high-melting polymorphic form of terfenadine (Form I) which comprises:

(a) forming a solution by dissolving terfenadine in acetone,

(b) diluting the solution with water, and

(c) collecting the crystallized product.

This process facilitates quality control functions associated with large scale production of terfenadine, and provides a substantially pure crystalline form of terfenadine which has a melting point of about 149°C to about 151°C.

Generally, the terfenadine solution is prepared by simply dissolving terfenadine in acetone by conventional and well known methods. Such methods include, but are not limited to, placing the acetone in a suitable container, adding the terfenadine to the acetone and then stirring, shaking, or swirling the container until the terfenadine is dissolved. Typically, the terfenadine dissolved will be substantially free of impurities, and the acetone used will have been filtered to clarity.

The solution can be formed at an ambient temperature, or it can be formed at elevated temperatures such as temperatures from about 25°C to about 56°C, to increase solubility although, the solution need not be heated for the purpose of this invention. Typically, the terfenadine/acetone solution will be heated, preferably to boiling.

The terfenadine/acetone solution typically will be highly concentrated, near the saturation point of solute in solvent, although the solution need not be saturated or substantially near saturation for purposes of this invention. For example, the weight to volume ratio of terfenadine to acetone can be from about 80 gm/l to about 2D0 gm/l, preferably from 95 gm/l to about 160 gm/l. Weight to volume ratios outside of the illustrative ranges above are contemplated as being within the scope of this invention.

Once terfenadine has been dissolved in acetone, the solution is diluted with water. Such dilution can be accomplished by standard and well known procedures. Such procedures include, but are not limited to, pouring the water into the solution all at once, or adding the water to the solution gradually or in several separate portions, each with stirring, shaking or any other means of mixing.

The amount of water which can be used to accomplish this dilution step is that amount which creates a volume to volume ratio of water to terfenadine/acetone solution of about 0.25:1 to about 1:1. The ratio of water to solution used generally will depend on the concentration of terfenadine in solution in the acetone. Typically, water will be added to the terfenadine/acetone solution until the solution becomes cloudy and crystallization begins, and this will involve a water/solution ratio of 1:1 or less, however, a volume/ volume ratio of greater than 1:1 may be used for the purpose of this invention.

Recrystallization of the product sufficient for collection will typically be accomplished within about 2 to about 20 hours after the solution is diluted with water. However the length of time required will vary depending on such factors as total volume of solution being processed, size of container, and degree of saturation of the terfenadine solution being processed, method of cooling the solution utilized, amount of product sought to be recovered, and presence or absence of stirring or other well-known methods that can be employed to enhance the recrystallization process. It is contemplated that recrystallization by utilization of this process will be accomplished within time periods outside the illustrative range noted above.

The diluted solution may, but, need not, be cooled for any desired period of time. For instance, cooling may be initiated as a matter of convenience to accelerate crystallization, or it may be maintained specifically for the period of time considered optimal for the desired purpose. Typically, the diluted solution will be cooled to a temperature of about 15°C to about 5°C for a period of about 2 hours to about 20 hours.

Methods known in the art may be used with the process of this invention for convenience or to enhance any aspect of this process. For example, the terfenadine solution may be seeded with one or more crystals of the desired polymorphic product prior to the initiation of product recrystallization.

The process of this invention is directed to the production of Form I of terfenadine which is the high-melting polymorphic form of terfenadine, characterized to melt at a temperature of about 149°C to about 151°C. This form is to be distinguished from Form II, which is the low-melting form and which melts at a temperature of about 144°C to about 147.5°C.

The product recovered by the process of this invention can range from an enriched product to a product which is IDD% pure. An enriched product is one that contains at least more than 50% of Form I of terfenadine. Generally, the product recovered by use of the process of this invention will be predominantly Form I, however, the degree of purity will depend on the conditions undertaken to perform the process. The degree of purity of the product will effect the exact melting point of the product. A product containing form I in less than 100% concentration may have a melting point lower than 149°C.

The following specific examples are presented to illustrate the process which is this invention, but they should not be construed as limiting the scope of this invention in any way.

EXAMPLE 1

PREPARATION OF TERFENADINE

A one-liter, three-neck flask equipped with a mechanical stirrer, a pressure-equalizing addition funnel and a reflux condenser fitted with nitrogen bubbler was charged with 72.8 g (0.149 mol) of terfenadone monohydrate and 320 ml absolute ethanol. The mixture was heated to reflux, a clear solution formed and a solution of 12.0 g (0.317 mol) of sodium borohydride in 83.2 g of 50% sodium hydroxide and 25.6 ml of water was added dropwise, with stirring, over a period of 1 hour. After the addition was complete, the reaction mixture was stirred at reflux for 2 hours. Terfenadine began to crystallize. The slurry was then diluted with 320 ml of water by adding the water at such a rate that the reaction remained at reflux. The slurry was then cooled slowly to room temperature. The solid was filtered from the slurry, washed with 100 ml of 50/50 (vol/vol) ethanol/water, washed with two 250 ml portions of deionized water, air dried, and vacuum dried at 60°C to give 68.2 g (97% yield) of terfenadine as a mixture of polymorphs.

EXAMPLE 2

PREPARATION OF FORM I FROM ACETONE SOLUTION

A 30 gallon reactor was charged with 8.56 kg of terfenadine from Example 1 and 88 liters of acetone. After the terfenadine had substantially dissolved, the solution was filtered to clarity. Then the solution was distilled to remove a total of 35 liters of acetone. The solution was diluted by gradually adding 18 liters of water to the clarified solution, with agitation. Upon crystallization, the diluted solution was cooled to about 5°C for 16 hours and the product was collected by filtration and dried in vacuo at 60°C to yield 7.6 kg (88.8%) of polymorphic form I of terfenadine. M.p. 147°C-149°C.

**Claims**

1. A process for producing a high-melting polymorphic form of terfenadine which comprises:
    (a) forming a solution by dissolving terfenadine in acetone,
    (b) diluting the solution with water, and
    (c) collecting the crystallized product.

2. A process according to Claim 1 wherein the volume to volume ratio of water to solution is 0.25:1 to 1:1.

3. A process according to Claim 1 wherein the weight to volume ratio of terfenadine to acetone is 80 gm/l to 200 gm/l.

4. A process according to Claim 2 wherein the weight to volume ratio of terfenadine to acetone is 80 gm/l to 200 gm/l.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | US-A-4 742 175  (T.G. FAWCETT et al.)<br>* the whole document * | 1 | C 07 D 211/22<br>C 07 B  63/00 |
| A | DE-A-2 303 306  (RICHARDSON-MERELL INC.)<br>* example 1 * | 1 | |
| P,A | EP-A-0 346 765  (GRUPPO LEPETIT SPA)<br>* the whole document * | 1 | |

| | TECHNICAL FIELDS SEARCHED (Int. Cl.5) |
|---|---|
| | C 07 D 211/00<br>C 07 B  63/00 |

The present search report has been drawn up for all claims

| Place of search | Date of completion of the search | Examiner |
|---|---|---|
| BERLIN | 06-08-1990 | VAN AMSTERDAM L.J.P. |